# EUROPEAN PATENT APPLICATION

(11) **EP 3 701 894 A1**
(43) Date of publication of application: **02.09.2020**
(21) Application number: 19194708.4
(22) Date of filing: 30.08.2019
(51) Int. Cl.: A61B 17/80, A61B 17/86

(54) **BONE PLATES AND ASSOCIATED SCREWS**

(30) Priority: 01.03.2019 EP 19160352
(71) Applicant: Stryker European Holdings I, LLC, Kalamazoo, MI 49002 (US)
(72) Inventor: DOMINIK, Robert, CH 1091 Grandvaux (CH)
(74) Representative: Röthinger, Rainer

(57) **Abstract**

A bone plate includes a bone-contacting surface, an upper surface opposite the bone-contacting surface, and a hole extending from the upper surface to the bone-contacting surface, the hole having an engagement region having a plurality of scalloped regions spaced around a circumference of the hole, adjacent scalloped regions being separated by a respective screw-engaging member that extends along a substantial portion of the depth of the engagement region, the screw-engaging member having a converging-diverging profile along the depth of the engagement region relative to a central axis of the hole.

## Description

### BACKGROUND OF THE INVENTION

The present disclosure relates to a bone plating system and methods of use thereof for the fixation of fractures of the bone, particularly in long bones, such as the femur, tibia, humerus and radius. More specifically, the present disclosure includes a bone plate system including a plate with screw holes adapted to receive both locking and non-locking screws and permit placement of those screws at various angles.

When a bone is damaged or fractured, bone plates are commonly attached to the outside surface of the damaged bone to stabilize the area and promote healing of the bone. Generally, the plates have a bone contacting side and a side facing away from the bone with a plurality of holes extending through the two surfaces. The holes are typically threaded for use with locking screws or non-threaded for use with non-locking, compression screws. Depending upon certain factors, such as bone quality and type, it may be more beneficial to utilize one type of screw over the other. Of course, the placement of dedicated locking and non-locking holes in the plate limits where such screws can be utilized. Moreover, certain types of holes, especially threaded holes, facilitate placement of a screw along only the axis of the hole.

There exists a need for holes that can receive both locking and compression screws in placed at various angles with respect to the axis of the hole to provide surgeons with additional options for securing the bone plate to the bone and thereby fixing the fracture.

### BRIEF SUMMARY OF THE INVENTION

The present disclosure generally relates to bone plating systems for receiving locking screws in polyaxial orientations as well as receiving non-locking, compression screws.

According to an embodiment of the present disclosure, a bone plate includes a bone-contacting surface, an upper surface opposite the bone-contacting surface, and a hole extending from the upper surface to the bone-contacting surface. The hole has an engagement region having a plurality of scalloped regions spaced around a circumference of the hole, adjacent scalloped regions being separated by a respective screw-engaging member that extends along a substantial portion of the depth of the engagement region, the screw-engaging member having a converging-diverging profile along the depth of the engagement region relative to a central axis of the hole.

In other embodiments, the bone plate may include a countersink extending from the upper surface to the engagement region of the hole. The engagement region may extend from the countersink to the bone-contacting surface. The screw-engaging member may extend from the countersink to the bone-contacting surface. The screw-engaging member may include a surface. The surface may extend the entire depth of the engagement region. The surface may define a rectangle along a majority of its length. The surface may be diamond shaped. The screw engaging member may include a first line and a second line extending in a direction of the depth of the engagement region, the surface being positioned between each of the first and the second line. The screw engaging member may include a line extending along at least a portion of the depth of the engagement region. The line may extend along a substantial portion of the depth of the engagement region. The line may extend the entirety of the depth of the engagement region. The hole may be configured to secure both of a locking screw and a non-locking screw within the plate. The engagement region may include at least eight scalloped regions.

In other embodiments, the bone plates may be included in a system for peri-prosthetic fracture repair. The system including a locking screw configured to be secured within the hole of the plate, wherein a head of the locking screw has a first hardness and the screw-engaging member of the hole of the bone plate has a second hardness that is softer than the first hardness. In other embodiment, the system may include a non-locking screw configured to be received within the hole of the plate.

### BRIEF DESCRIPTION OF THE DRAWINGS

Fig. 1 shows a top view of an illustrative bone plate according to an embodiment of the present disclosure;
Fig. 2 shows a perspective side view of the bone plate of Fig. 1;
Fig. 3 shows a cross-sectional view of a hole of a bone plate according to another embodiment of the present disclosure;
Fig. 4 is a top view of the hole of Fig. 3;
Fig. 5 is a cross-sectional view of the hole of Fig. 3 in conjunction with a locking bone screw;
Fig. 6 is a perspective side view of a non-locking screw for use with the hole of Fig. 3;
Fig. 7 is a cross-sectional view of an alternative embodiment of a hole according to an embodiment of the present disclosure;
Fig. 8 is a cross-sectional view of an alternative embodiment of a hole according to an embodiment of the present disclosure;
Fig. 9 is a top view of the hole of Fig. 8;
Fig. 10 is a cross-sectional view of an alternative embodiment of a hole according to an embodiment of the present disclosure;
Fig. 11 is a top view of the hole of Fig. 10;
Fig. 12 is a top view of a hole of a bone plate according to another embodiment of the present disclosure;
Fig. 13 is an enlarged view of the hole of Fig. 12;
Fig. 14 is a top view of a hole of a bone plate according to another embodiment of the present disclosure;
Fig. 15 is an enlarged view of the hole of Fig. 14;
Fig. 16 is a top view of a hole of a bone plate according to another embodiment of the present disclosure; and
Fig. 17 is an enlarged view of the hole of Fig. 16.

### DETAILED DESCRIPTION

As used herein unless stated otherwise, the term "anterior" means toward the front part of the body, and the term "posterior" means toward the back part of the body. When referring to specific directions in the following discussion of a certain device, the terms "proximal" and "distal" are to be understood in regard to the device's orientation and position during exemplary application to human body. Thus, the term "proximal" means closer to the operator or in a direction toward the operator, and the term "distal" means more distant from the operator or in a direction away from the operator. In addition, the terms "about," "generally," and "substantially" are intended to mean that deviations from absolute are included within the scope of the term so modified.

Additionally, certain of the embodiments include similar features, for which similar reference numerals are utilized, albeit within a different 100-series of numbers.

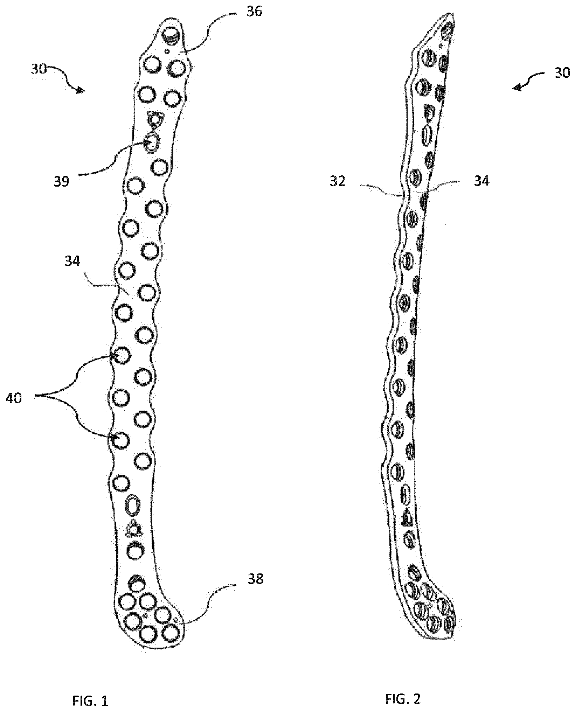

Referring to Figs. 1-2, an embodiment of a polyaxial screw system for the fixation of bone fractures according to the present disclosure is shown. The system includes illustrative bone plate 30 defining plate holes 40 adapted to receive locking and/or non-locking screws. Generally, embodiments of the present disclosure include bone plates that may be similar to plate 30, although each embodiment includes a different feature that enables polyaxial locking of screws within holes 40, as will be described in further detail below. Further, each of the bone plates of the present disclosure may receive locking screws, compression screws, or a combination of both in the plurality of polyaxial holes 40. Although one type and/or shape of plate 30 is shown herein, and such plate may indeed be novel and unobvious in view of the prior art, it is to be understood that plates according to the present invention can be designed for use on various bones and thusly can be any configuration necessary for such use. Indeed, certain plate sizes and shapes are well known for certain applications and the different hole configurations noted below can be utilized therewith.

In the illustrated embodiment, plate 30 includes bone-contacting surface 32 and upper surface 34 opposite the bone-contacting surface. In an implanted configuration, bone-contacting surface 32 is positioned on the bone and upper surface 34 is substantially opposite to the bone-contacting surface. Generally, throughout the various embodiments of the present disclosure, the term "inferior" means toward the bone-contacting surface and the term "superior" means toward the upper surface.

Plate 30 defines a length extending from first end 36 to second end 38. In the embodiment shown, plate 30 has a substantially constant plate thickness. First end 36 is anatomically formed to conform to the trochanter region of the femur. In particular, in an implanted state, first end 36 conforms to the convex surface of the femur in the region of the greater trochanter. Second end 38 is anatomically formed to conform to the condyle region, in particular the convex surface of the femur in the region of the medial condyle. As such, plate 30 provides conformity both in the trochanter region and in the condyle region of the femur, without requiring intense contouring when fixing the plate to the femur. In another embodiment, the plate thickness may vary along the length of the plate. Plate 30 may be formed from a single piece of rigid material, such as stainless steel, titanium and its alloys.

As shown in Figs. 1 and 2, plate 30 may include at least one oblong hole 39 extending from bone-contacting surface 32 to upper surface 34 for receiving a non-locking screw. Oblong hole 39 may have a length extending in the same direction as the length of plate 30 and preferably acts in conjunction with the non-locking screw to compress a fracture, such as a peri-prosthetic fracture. Plate 30 also includes a plurality of holes 40 extending from bone-contacting surface 32 to upper surface 34. Holes 40 are circular, although in other embodiments the holes may have a different shape. Holes 40 are configured to accept locking and non-locking screws and at varying angles with respect to an axis formed through the hole. In other words, the holes include a polyaxial locking feature. Generally, embodiments of the present disclosure include bone plates that may be similar to plate 30, although certain embodiments include different features that enable polyaxial locking of screws within holes 40, as will be described in further detail below. Although holes 40 are described herein with connection to plate 30, the holes may be employed on a bone plate having any shape, and the bone plate shown in Figs. 1-2 is not meant to be limiting.

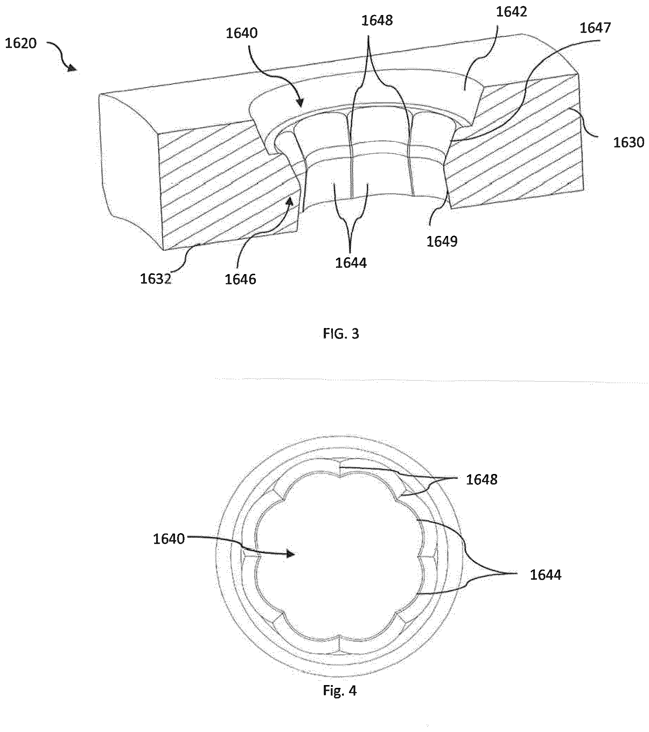

Bone plating system 1620 includes plate 1630 with holes 1640 for receiving locking screws 1660 at polyaxial orientations. As shown in Fig. 3, each hole 1640 includes countersink 1642 and engagement region 1646 extending from the countersink to bone-contacting surface 1632.

As shown in Fig. 4, from the top view, hole 1640 has a flower-shape configuration formed from a plurality of spaced apart scalloped regions 1644 and screw-engaging members 1648 which are spaced apart about the central axis of hole 1640. Each screw-engaging member 1648 extends in a direction from countersink 1642 toward bone-contacting surface 1632 and separates adjacent scalloped regions 1644. In the illustrated embodiment, hole 1640 includes eight equally spaced apart screw-engaging members 1648 and scalloped regions 1644. In this embodiment, each screw-engaging member 1648 defines a surface defining a width as it extends in a direction parallel to the central axis of the hole. As shown in Fig. 3, engagement region 1646 has a converging-diverging profile, in which an upper portion 1647 tapers toward the central axis of hole 1640 and a lower portion 1649 tapers away from the central axis of the hole 1640. Although shown with a specific number of scalloped regions and engaging members, it is to be understood that different embodiments according to the present invention may exhibit any number of each element.

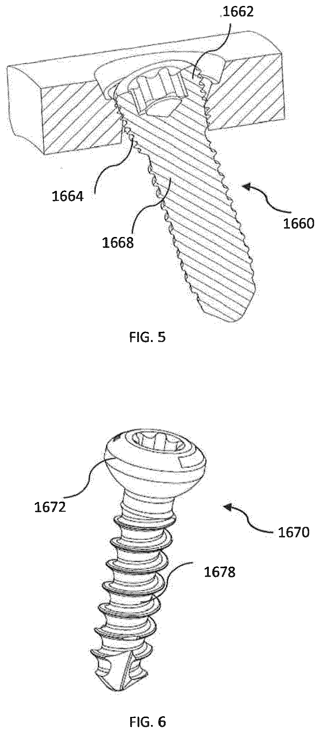

Fig. 5 shows locking screw 1660 positioned within hole 1640. Locking screw 1660 includes a conically shaped head 1662 having threads 1664 and a threaded shaft 1668. When locking screw 1660 is positioned within hole 1640, threads 1664 engage screw-engaging members 1648 of hole 1640 by plastically deforming the screw-engaging members 1648 to secure the locking screw 1660 to the bone plate 1630 at variable angles. Other screw designs can also be utilized, for instance, screws with curved or rounded heads.

Holes 1640 are configured to receive non-locking screws 1670 in addition to locking screws 1660. As shown in Fig. 6 non-locking screw 1670 has a substantially rounded head 1672 and a threaded shaft 1678. This type of screw may or may not interact with any of the elements of the screw hole in which they are placed.

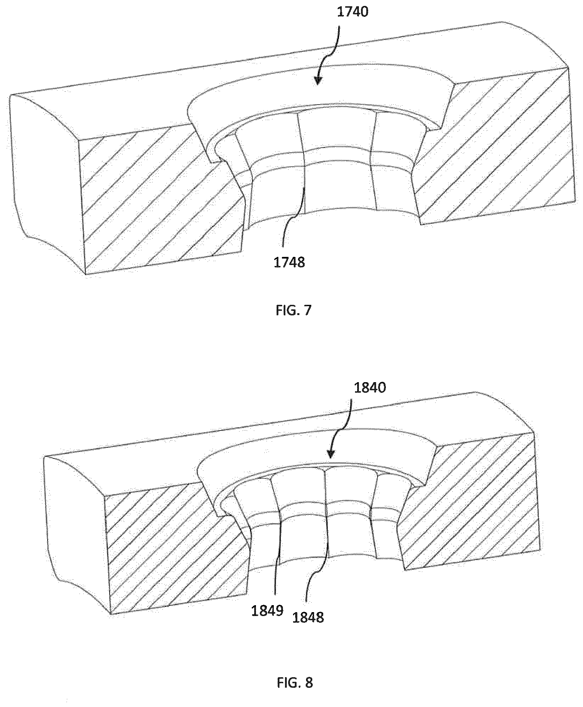

Fig. 7 shows hole 1740 which is identical to hole 1640 except that each screw-engaging members 1748 defines a line rather than a surface as in hole 1640.

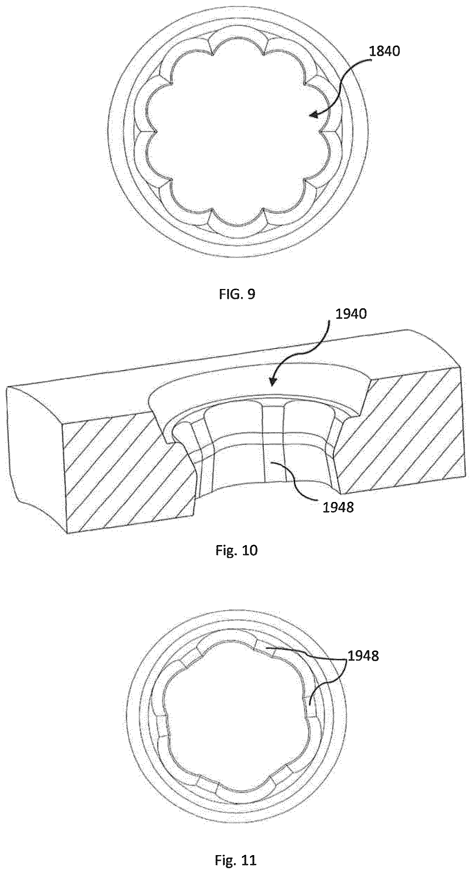

Figs. 8 and 9 show hole 1840 which is an alternative embodiment of holes 1640 and 1740. Each screw-engaging member 1848 includes a line that transitions into a diamond-shaped surface 1849. Locking screw 1660 engages and plastically deforms the diamond-shaped surface 1849 to secure the screw to the hole. Further, hole 1840 differs from hole 1640 in that it includes ten equally spaced apart screw-engaging members 1848, as shown in Fig. 9.

Figs. 10 and 11 show hole 1940 which is similar to hole 1640 except that screw-engaging members 1948 of hole 1940 form a wider surface with a substantially constant width along the length of the surface of each member 1948.

During insertion of a locking screw in any of holes 1640, 1740, 1840, and 1940, a locking screw, such as locking screw 1660, is torqued within the hole. As the screw head rotates, the threads 1664 of screw head 1662 engage at least some of the screw-engaging members 1648 to form an interference connection between the screw-engaging members and the screw head which causes the screw-engaging members to plastically deform. This connection results in the locking of the screw to the plate and prevents subsequent loosening of the screw.

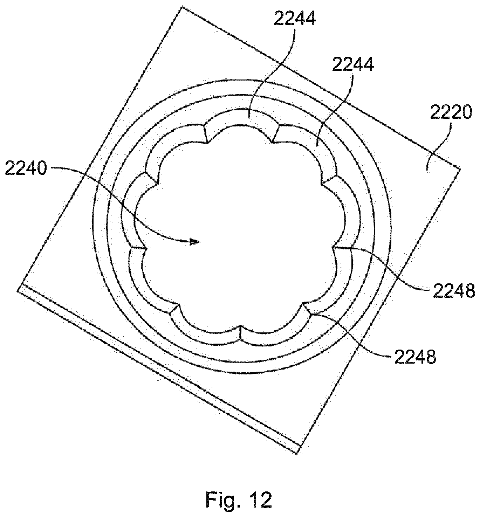

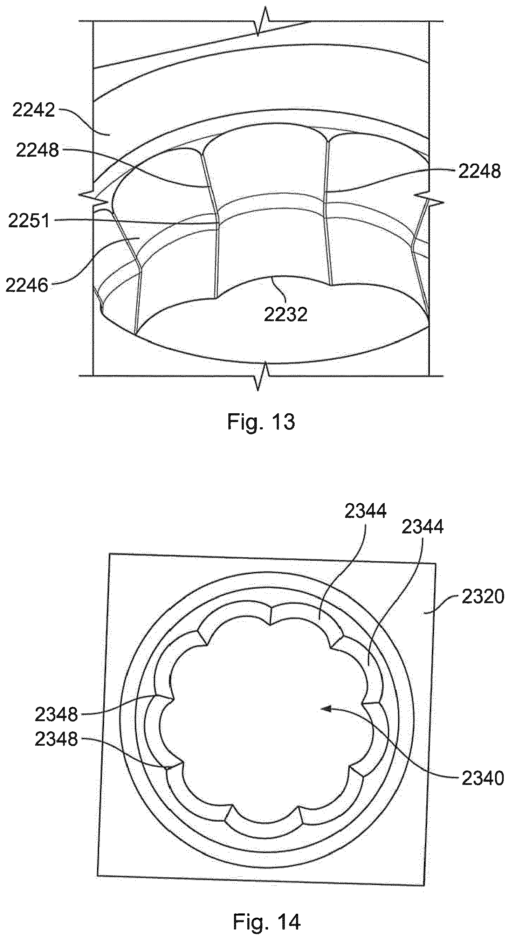

Figs. 12 and 13 show hole 2240 of bone plate 2220 that is an alternative embodiment of a bone plate according to the present disclosure. Hole 2240 is configured to receive locking screws 1660 at polyaxial orientations within the hole and/or compression screws 2270. Holes 2240 are substantially similar to holes 1640, described above, except that the hole includes nine scalloped regions 2244 and nine screw-engaging members 2248. Each hole 2240 includes countersink 2242 extending from upper surface 2234 to engagement region 2246. And, each engagement region 2246 extends from countersink 2242 to bone-contacting surface 2232. Adjacent scalloped regions 2244 are separated by and intersect at a respective one of the plurality of screw engaging members 2248, which are also spaced apart around the circumference of the hole.

Each screw-engaging member 2248 extends in a direction from countersink 2242 toward bone-contacting surface 2232, *e.g.* along a depth of the engagement region 2246. Each screw-engaging member 2248 extends along a substantial portion of the depth of the engagement region 2246, e.g. extends along greater than 50% of the depth of the engagement region. Each screw-engaging member 2248 may extend along the entirety of the depth of the engagement region and may extend from countersink 2242 to bone-contacting surface 2232.

In this embodiment, each screw-engaging member 2248 includes a surface that defines a length, measured in the direction from the upper surface to the bone-contacting surface, and a width, measured in the direction of the circumference of the hole, as shown in detail in Fig. 13. The surface has a generally rectangular shape, with the length of the surface being greater than the width. As shown in Fig. 13, engagement region 2246 has a converging-diverging profile along a depth of the engagement region relative to the central hole axis, in which an upper portion of the engagement region 2246 tapers from countersink 2242 toward the central hole axis and a lower portion tapers away from the central hole axis. The upper and lower portions meet at a transition point 2251, which extends farthest radially inward toward the central hole axis.

In use, bone-contacting surface 2232 is positioned on the damaged bone. Locking screw 1660 is inserted into hole 2240 with head 1662 positioned within and surrounded by hole 2240 and shaft 1668 extending through bone-contacting surface 2232 and into the bone. Locking screw 1660 may be inserted into the hole within a cone of 30 degrees with respect to an axis of the hole. With locking screw 1660 within hole 2240, the screw may be torqued. Due to the conical shape of head 1662, the head applies radial forces to the bone plate 2220 so that the threads of the head engage screw-engaging members 2248 of hole 2240, which results in a form fit. In this regard, plate 2220 may have a plurality of holes 2240, and each locking screw 1660 may be placed at a same or different angle with respect to the axes of the bone holes.

Alternatively, compression screw 1670 may be inserted into hole 2240 and secured to the bone. The head of such screw may rest within countersink 2242, without engaging screw-engaging members 2248. As such, plate 2220 can accommodate locking screws 1660, in the manner described above, as well as compression screws 1670 in holes 2240. The plurality of the holes 2240 of plate 2220 may include all locking screws 1660, or all compression screws 1670, or a combination of both.

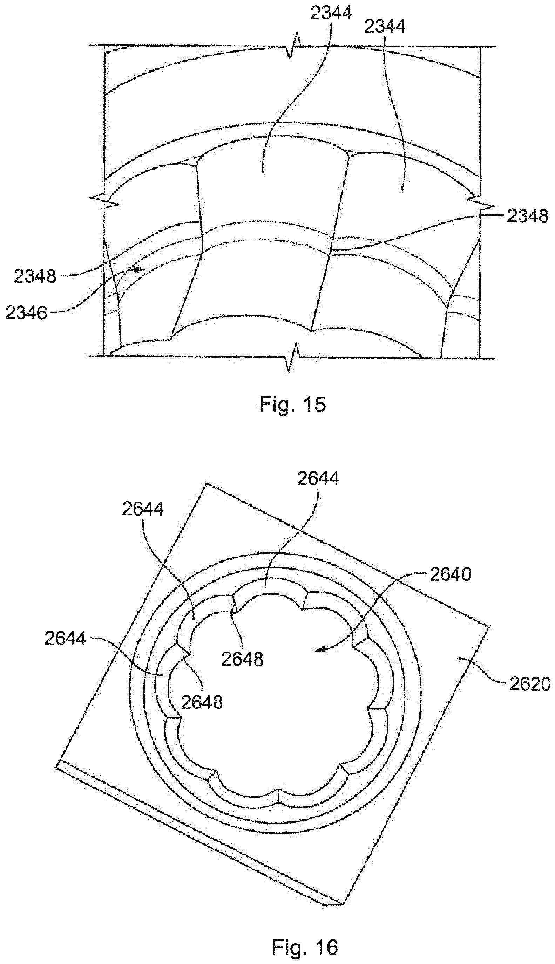

Figs. 14 and 15 show bone plate 2320, which is identical in most respects to holes 1740, shown in Fig. 7 above, except that plate 2320 includes nine scalloped regions 2344 and nine screw-engaging members 2348. Fig. 15 shows an enlarged view of scalloped screw-engaging members 2348 extending further radially inward toward the central hole axis than the scalloped regions 2344. Each screw-engaging member 2348 includes a line, e.g. connection of points with negligible width. In this embodiment, screw-engaging members 2348 each extends along a substantial portion of the engagement region 2346, e.g. extends along greater than 50% of the engagement region. And, in the illustrated embodiment, screw-engaging member, e.g. line, extends from the countersink to the bone-contacting surface and has a converging-diverging profile in the direction of the depth of the engagement region 2344 relative to the central hole axis.

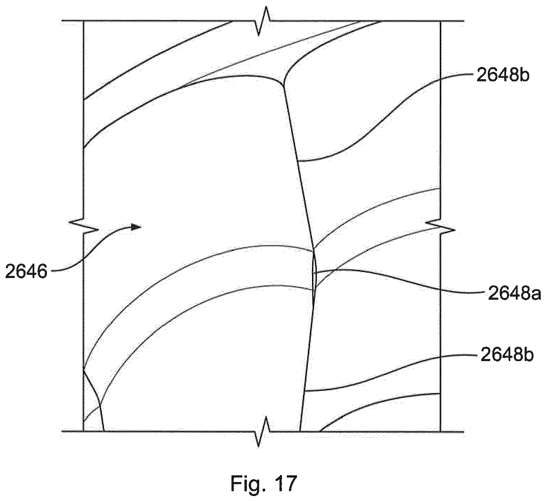

Figs. 16 and 17 show hole 2640 which is an alternative embodiment of hole 1840, in that hole 2640 includes nine scalloped regions 2644 and nine screw-engaging members 2648 that extend along a substantial portion of the depth of engagement region 2646.

Fig. 17 shows an enlarged view of screw-engaging members 2648, which is formed of lines 2648b with substantially diamond-shaped surface 2648b positioned between and connecting each of the first and the second line. Thus, two lines 2648b and one single diamond-shaped surface 2648a form one single screw-engaging member, and each extends along a portion of the depth of the engagement region. of the lines and the Diamond-shaped surface 2648b is positioned at the transition point of the converging-diverging profile of a respective screw-engaging member, e.g. where the screw-engaging member transitions from converging radially toward the central hole axis to diverging radially away from the central hole axis. In use, locking screw 1660 engages and plastically deforms the diamond-shaped surface 2648a to secure the screw to the hole.

During insertion of a locking screw in any of holes 1640, 1740, 1840, 1940 2240, 2340 and 2640, the locking screw, such as locking screw 1660, is torqued within the hole. As the screw head rotates, the threads 1664 of screw head 1662 engage at least some of the screw-engaging members 1648 to form an interference connection between the screw-engaging members and the screw head which causes the screw-engaging members to plastically deform. This connection results in the locking of the screw to the plate and prevents subsequent loosening of the screw.

The plates described above are formed from a single piece of rigid material, such as stainless steel, titanium and its alloys. In other examples, the plates may be formed from other biocompatible materials including bioceramics and polymers. In some instances, the locking screws may be formed of cobalt chromium and may be harder than the material of the plate. In other instances the screws may be formed of stainless steel, titanium and its alloys, etc. It is contemplated to form the plates utilizing any known manufacturing method, including molding, milling and additive manufacturing.

It will be appreciated that the features described in connection with individual embodiments may be shared with others of the described embodiments. Further, individual plates may include one or more the described hole designs.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

## Claims

1. A bone plate comprising:
a bone-contacting surface;
an upper surface opposite the bone-contacting surface; and
a hole extending from the upper surface to the bone-contacting surface, the hole having an engagement region having a plurality of scalloped regions spaced around a circumference of the hole, adjacent scalloped regions being separated by a respective screw-engaging member that extends along a substantial portion of the depth of the engagement region, the screw-engaging member having a converging-diverging profile along the depth of the engagement region relative to a central axis of the hole.

2. The bone plate of claim 1, further comprising a countersink extending from the upper surface to the engagement region of the hole.

3. The bone plate of claim 2, wherein the engagement region extends from the countersink to the bone-contacting surface.

4. The bone plate of claim 2 or 3, wherein the screw-engaging member extends from the countersink to the bone-contacting surface.

5. The bone plate of any of the preceding claims, wherein the screw-engaging member includes a surface.

6. The bone plate of claim 5, wherein the surface extends the entire depth of the engagement region.

7. The bone plate of claim 5 or 6, wherein the surface defines a rectangle along a majority of its length.

8. The bone plate of claim 5 or 6, wherein the surface is diamond shaped.

9. The bone plate of any of claims 5 to 8, wherein the screw engaging member includes a first line and a second line extending in a direction of the depth of the engagement region, the surface being positioned between each of the first and the second line.

10. The bone plate of any of claims 1 to 4, wherein the screw engaging member includes a line extending along at least a portion of the depth of the engagement region.

11. The bone plate of claim 10, wherein the line extends along the entirety of the depth of the engagement region.

12. The bone plate of any of the preceding claims, wherein the hole is configured to secure both of a locking screw and a non-locking screw within the plate.

13. The bone plate of any of the preceding claims, wherein the engagement region includes at least eight scalloped regions.

14. A system for peri-prosthetic repair comprising:
the bone plate of any of the preceding claims; and
a locking screw configured to be secured within the hole of the plate, wherein a head of the locking screw has a first hardness and the screw-engaging member of the hole of the bone plate has a second hardness that is softer than the first hardness.

15. The system of claim 14, further comprising a non-locking screw configured to be received within the hole of the plate.
